Europäisches Patentamt

European Patent Office

Office européen des brevets

(19)

(11) Veröffentlichungsnummer : **0 148 293**
**B1**

(12)        # EUROPÄISCHE PATENTSCHRIFT

(45) Veröffentlichungstag der Patentschrift :
05.04.89

(51) Int. Cl.⁴ : **C 07 D323/06**

(21) Anmeldenummer : **84100101.9**

(22) Anmeldetag : **07.01.84**

(54) **Verfahren zur Herstellung von Trioxan.**

(43) Veröffentlichungstag der Anmeldung :
**17.07.85 Patentblatt 85/29**

(45) Bekanntmachung des Hinweises auf die Patenterteilung : **05.04.89 Patentblatt 89/14**

(84) Benannte Vertragsstaaten :
**BE FR GB IT NL SE**

(56) Entgegenhaltungen :
**GB—A— 1 012 372**
**GB—A— 1 130 513**
**PATENTS ABSTRACTS OF JAPAN, Band 8, Nr. 34 (C-**
**210)(1471), 15. Februar 1984; & JP - A - 58 198482**
**(ASAHI KASEI KOGYO K.K.) 18.11.1983**
**SOVIET INVENTIONS ILLUSTRATED, Sektion Chemi-**
**cal, Derwent Publications Ltd., Woche E03, 3. März**
**1982, Sektion C, E13; & SU - A - 819 104 (PAKULIN V V)**
**06.05.1981**
**PATENTS ABSTRACTS OF JAPAN, Band 5, Nr. 41 (C-**
**47)(713), 18. März 1981; & JP - A - 55 164680 (MITSU-**
**BISHI GAS KAGAKU K.K.) 22.12.1980**

(73) Patentinhaber : **Küppenbender, Herbert**
**Lenzhahner Weg 36**
**D-6272 Niedernhausen (DE)**

**Rauxloh, Bernhard**
**Herderstrasse 2**
**D-6000 Frankfurt a.M. 1 (DE)**

(72) Erfinder : **Küppenbender, Herbert**
**Lenzhahner Weg 36**
**D-6272 Niedernhausen (DE)**
Erfinder : **Rauxloh, Bernhard**
**Herderstrasse 2**
**D-6000 Frankfurt a.M. 1 (DE)**

(74) Vertreter : **Lotterhos, Hans Walter, Dr.-Ing.**
**Lichtensteinstrasse 3**
**D-6000 Frankfurt am Main 1 (DE)**

**Beschreibung**

Zur Herstellung von Trioxan wird gewöhnlich von den im Handel erhältlichen, lagerstabilen, etwa 30-40 %igen Formalinlösungen ausgegangen, die entweder nach dem Formoxverfahren, durch Oxadation von Methanol mit einem Überschuß an Luft in Gegenwart von modifiziertem Eisenoxyd/Molybdänoxyd-Katalysatoren im Temperaturbereich von 250-400 °C oder nach dem Silberkontaktverfahren durch partielle Oxydation und Dehydrierung von Methanol mit einem Unterschuß an Luft in Gegenwart von kristallinem Silber oder Silber-Netzen im Temperaturbereich von etwa 600-720 °C erhalten werden.

Die so erhaltenen Formalinlösungen werden bei den bekannten Verfahren zur Trioxanherstellung zunächst auf einen Formaldehydgehalt von etwa 50-70 Gew.% aufkonzentriert und dann im Trioxanreaktor in Gegenwart eines Säurekatalysators zum Sieden erhitzt. Hierbei findet eine Trimerisierung des Formaldehyds zum Trioxan statt, die in dem wäßrigsauren Reaktionsmedium zu einem weit auf der Seite des monomeren Formaldehyds liegenden Gleichgewicht führt. Da jedoch von den im Reaktionsmedium enthaltenen Komponenten : Trioxan, Formaldehyd und Wasser, das Trioxan die größte Flüchtigkeit aufweist, findet in dem bei dem Abdestillieren aus dem Trioxanreaktor austretenden Synthesedampfgegenüber dem flüssigen Reaktionsgemisch — eine Anreicherung an Trioxan und damit eine Störung des Gleichgewichts in der flüssigen Phase unter weiterer Trioxanbildung statt.

Das im Synthesedampf enthaltene Trioxan wird bei den bekannten Verfahren durch eine auf den Trioxanreaktor aufgesetzte Rektifizierkolonne oder in einer nachgeschalteten Kolonne weiter angereichert. Aus dem so erhaltenen Konzentrat wird das Trioxan mit einem mit Wasser nicht mischbaren inerten Lösungsmittel, wie Methylenchlorid, Äthylenchlorid, Benzol, extrahiert, während die wäßrige, Formaldehyd-enthaltende Phase in den Trioxanreaktor zurückgeleitet wird. Der Extrakt wird dann durch fraktionierte Destillation vom Extraktionsmittel befreit und das erhaltene rohe Trioxan der Reindestillation unterworfen.

Als Säurekatalysator werden Mineralsäuren, z. B. Schwefelsäure, Phosphorsäure, Ionenaustauscher, die in einer Konzentration bis 25 % vorliegen, angewandt, und die saure Reaktionslösung wird mittels eines Umlaufverdampfers zum Sieden erhitzt. Bei dieser Verfahrensführung beträgt die Trioxankonzentration in dem den Reaktor verlassenden Synthesedampf 18 Gew.%, was bei einer Formaldehydkonzentration im Reaktoreinlauf von 60 % einem Formaldehydumsatz pro Reaktordurchgang von 30 % entspricht.

Aus der DE-A-15 93 990 ist bekannt, gasförmigen Formaldehyd, der durch Pyrolyse von Paraformaldehyd oder durch Erhitzen einer Formalinlösung erhalten worden ist, in der Gasphase in Gegenwart eines speziellen Harzaustauschers in der Trimerisierungskolonne in Trioxan überzuführen. Aus dem so erhaltenen Gemisch von Trioxan und Formaldehyd wird in einem Abscheider das Trioxan in fester Form abgeschieden und isoliert, während der gasförmige Formaldehyd in die Trimerisierungskolonne zurückgeführt wird.

Aus der JP-A-58 198 482 (Pat. Abstr. of Japan, 8, Nr. 34) ist es bekannt, zur Herstellung von sehr reinem Trioxan gasförmigen, wasserfreien Formaldehyd in einen Trioxanreaktor einzuleiten, in dem eine Formalinlösung, die einen sauren Katalysator enthält, zum Sieden erhitzt wird und aus dem dabei erhaltenen Synthesedampf — nach Durchlaufen der Destillationszone — das Trioxan mit einem wasserunlöslichen organischen Lösungsmittel, das mit Wasser ein Azeotrop bildet, durch Extraktion zu isolieren.

Aus der GB-A-1 130 513 ist es bekannt, zur Senkung des Energieverbrauchs bei der Trioxanherstellung, das heiße Formaldehyd-Wasserdampf-Gemisch, das nach dem Aufkonzentrieren einer etwa 30 %igen Formalin-Lösung in einer Druckkolonne erhalten wird, in den Trioxanreaktor einzuleiten, in dem — unter Nutzung der mit dem Formaldehyddampf eingeführten Wärme — der Formaldehyd in Gegenwart eines sauren Katalysators in Trioxan übergeführt wird.

Bei beiden Verfahren wird das in den Trioxanreaktor eingeleitete Formaldehydgas sofort von der heißen Reaktorlösung absorbiert.

Die Erfindung betrifft ein Verfahren zur Herstellung von Trioxan aus gasförmigem, erhitztem Formaldehyd durch Trimerisierung und Isolation des Trioxans aus dem Kondensat durch Extraktion mit einem mit Wasser nicht mischbaren inerten Lösungsmittel und Destillation in Gegenwart eines Säurekatalysators, das dadurch gekennzeichnet ist, daß man einen heißen, Formaldehyd enthaltenden Gasstrom in den unteren Teil des Trioxanreaktors einleitet, wobei die in dem Reaktor enthaltene etwa 30 bis 70 Gew.%ige Formalinlösung, die einen Säurekatalysator enthält und einen pH-Wert aufweist, der dem von 2 bis 25 Gew.% Schwefelsäure entspricht, auf den Siedepunkt erhitzt und gut durchgemischt wird, den aus dem Trioxanreaktor austretenden Trioxan, Formaldehyd und Wasser enthaltenden Synthesedampf an einem, dem Trioxanreaktor unmittelbar nachgeordneten Dephlegmator, der mit Wasser auf 90 °C gekühlt wird, in eine an Formaldehyd reiche Phase, die in den Trioxanreaktor zurückgeführt wird, und in eine an Trioxan reiche Phase, die kondensiert wird, unterteilt, und das Trioxan enthaltende Kondensat weiterverarbeitet.

Bei dieser Verfahrensführung wird eine überraschend hohe Trioxankonzentration von etwa 32 Gew.% im Synthesedampf erhalten, was bei einer Formaldehydkonzentration in der Reaktorzuführung von etwa 62 % einem Formaldehydumsatz pro Reaktordurchgang von etwa 52 % entspricht.

Als Säurekatalysator können in dem Verfahren der Erfindung Mineralsäuren, wie Schwefelsäure und/oder Phosphorsäure, oder sonstige Lewissäuren verwendet werden, wobei den Mineralsäuren, insbesondere der Schwefelsäure, der Vorzug gegeben wird. Der pH-Wert der Lösung entspricht dem einer 2-25, vorzugsweise 8-15 %igen Schwefelsäure.

Als Formaldehyd enthaltenden Gasstrom, wird das bei der katalytischen Methanoloxydation erhaltene heiße Formaldehydgas verwendet, das sowohl von mitgeführter Luft als auch von den bei der Methanoloxydation gebildeten Nebenprodukten begleitet sein kann. Gewünschtenfalls kann der Formaldehydgasstrom vor dem Einleiten in den Trioxanreaktor von unerwünschten Begleitstoffen befreit werden.

Als Lieferanten für den gasförmigen Formaldehyd kann sowohl der aus einem Silberkontaktverfahren als auch der aus dem Formox-Verfahren stammende Formaldehydgasstrom oder auch eine Mischung aus Formaldehyd, sauerstoff-armer Luft und Wasserdampf verwendet werden.

Bei Durchführung des Verfahrens der Erfindung wird der aus dem Formaldehydreaktor austretende Formaldehydgasstrom auf eine Temperatur im Bereich von 110-140 °C gekühlt und in den Bodenteil des Trioxanreaktors geleitet. Die mit dem Formaldehyddampf in den Trioxanreaktor eingebrachte Wärmeenergie reicht aus, um die saure Formalinlösung auf Reaktionstemperatur zu halten, so daß der bislang zum Erhitzen der Reaktorflüssigkeit verwendete Umlaufverdampfer entfällt. Durch Maßnahmen, wie Einleiten des heißen Formaldehydgasstroms in den Bodenteil des Reaktors und die Anwesenheit von Begleitgasen in dem Formaldehyd enthaltenden gasstrom wird eine gute Durchmischung des Reaktorinhalts erreicht.

Aus diesem Grund ist der gemäß Erfindung verwendete Trioxanreaktor zweckmäßig mit Einrichtungen, wie Füllkörper tragenden Verteilerböden, ausgestattet, die die bereits mit der Einleitung der Gasmischung in die saure Formalinlösung erzeugte Durchmischung der Lösung verstärken.

Der gemäß Erfindung verwendete Trioxanreaktor ist mit einem Dephlegmator verbunden, der mit temperiertem Wasser von 90 °C gekühlt wird. In dem Dephlegmator tritt eine Teilkondensation ein, wobei die an Formaldehyd reiche Phase in den Reaktor zurückfließt, während die an Trioxan reiche Phase, wie gefunden wurde, 32 Gew.% Trioxan aufweist, in einen Absorber geleitet wird. Das hier anfallende Kondensat kann auf Grund der hohen Trioxankonzentration direkt in eine Extraktionskolonne geleitet werden, in der das Trioxan aus der wäßrigen Lösung mit einem inerten, mit Wasser nicht mischbaren Lösungsmittel, wie Benzol, Äthylenchlorid, Methylenchlorid, extrahiert wird. Die bei der Extraktion erhaltene wäßrige Phase kann, nach dem Entfernen von Methanol und Aufkonzentrieren auf einen Gehalt von 40-60 Gew.% Formaldehyd, in Gasform oder flüssiger Form wieder in den Prozeß zurückgeführt oder bei Verwendungsmöglichkeit ohne Aufkonzentrierung als 40 %iges Formalin weiterverarbeitet werden.

Das nach der Entfernung des inerten Lösungsmittels erhaltene Rohtrioxan kann zur Reinigung einer Destillation oder einer Kristallisation unterworfen werden.

Wird als Formaldehydlieferant der aus dem Formox-Reaktor stammende Gasstrom verwendet, wird im Trioxanreaktor eine Formalinlösung verwendet, die zweckmäßig eine Formaldehydkonzentration von 42-65, vorzugsweise von 50 Gew.% aufweist.

Wird als Formaldehydlieferant der aus einem Silberkontakverfahren stammende Gasstrom verwendet, wird im Trioxanreaktor eine Formalinlösung verwendet, die zweckmäßig eine Formaldehydkonzentration von 30-45 Gew.% aufweist.

Die mit dem Verfahren der Erfindung erzielten Vorteile bestehen einerseits in einer überraschend hohen Trioxankonzentration im Synthesedampf und damit in einer unerwartet starken Erhöhung des Formaldehydumsatzes pro Reaktordurchgang und andererseits darin, daß mit dem Einführen des aus dem Formaldehydreaktor kommenden Formaldehydgasstroms in den Trioxanreaktor, die Herstellung der handelsüblichen, 30-42 Gew.%igen Formalinlösungen und deren Aufkonzentrieren auf den für die Trioxanreaktion notwendigen Formaldehydgehalt überflüssig wird.

Dies ist nicht nur mit der Einsparung einer ganzen Verfahrensstufe, der Formaldehydabsorption unter Bildung der niederprozentigen Formalinlösungen des Handels, sondern auch mit einer beträchtlichen Einsparung an Energie — der zum Aufkonzentrieren der niederprozentigen Formalinlösungen des Handels vor der Trioxanreaktion benötigten Wärmeenergie — verbunden.

Nachfolgend wird die Erfindung an einer beigefügten Zeichnung näher beschrieben, gemäß welcher die aus dem Formaldehydreaktor a) austretenden Formaldehyd enthaltenden Reaktionsgase nach Abkühlung im Reaktionsgaskühler b) auf eine Temperatur im Bereich von 110-140 °C in den Bodenteil des Trioxanreaktors c) eingeleitet werden, der eine auf Siedetemperatur befindliche saure Formaldehydlösung mit einem Formaldehydgehalt von 30-70 Gew.% enthält und mit Einrichtungen ausgestattet ist, die die Durchmischung des eingeleiteten Formaldehydgasstroms mit der Katalysator enthaltenden Reaktorlösung intensivieren und damit die Trimerisierung des Formaldehyds zum Trioxan begünstigen.

Der aus dem Trioxanreaktor c) austretende Synthesedampf wird dann an dem Dephlegmator d) teilkondensiert, wobei die an Formaldehyd reiche Phase in den Trioxanreaktor c) zurückgeleitet wird. Die damit in den Trioxanreaktor c) zurückgeführte Flüssigkeitsmenge einschließlich der mit dem gasförmigen Formaldehyd eingeführten Wassermenge reicht aus, um die während der Trioxanreaktion verdampfende Flüssigkeitsmenge zu kompensieren. Die an Trioxan reiche Phase wird in dem Absorber e) kondensiert. Das hier anfallende Kondensat kann auf Grund der hohen Trioxankonzentration direkt in die Extraktionskolonne f) geleitet werden, in der aus dem wäßrigen Konzentrat mit inertem, mit Wasser nicht mischbaren Lösungsmitteln (Benzol, Äthylenchlorid, Methylenchlorid) das Trioxan herausgelöst wird, während die wäßrige Formaldehydlösung gegebenenfalls nach Befreiung von Methanol in g) und Aufkonzentrierung in j) auf eine Formaldehydkonzentration von 60 % gasförmig vor dem Trioxanreaktor c) in den Prozeß zurückgeleitet wird.

Die aus der Extraktionskolonne f) austretende wäßrige, 40 %ige Formalinlösung kann entweder zur weiteren Verarbeitung aus dem Prozeß herausgeführt oder nach Befreiung von Methanol in g) vor dem Formaldehydreaktor a) in den Prozeß zurückgeleitet werden.

Das aus der Extraktionskolonne f) austretende Trioxan-Lösungsmittelgemisch wird nach Neutralisation durch fraktionierte Destillation in h) vom Lösungsmittel getrennt und das erhaltene Trioxan in i) durch eine Reindestillation oder Kristallisation gereinigt.

Das Verfahren der Erfindung eignet sich insbesondere zur kontinuierlichen Verfahrensführung.

Das Verfahren der Erfindung soll durch das nachstehende Beispiel näher erläutert werden.

Beispiel

Ein gasförmiger Aldehydstrom, der
93 % sauerstoffarme Luft (etwa 10 %)
4,4 % Formaldehyd und
2,6 % Wasserdampf
enthielt, wurde mit einer Temperatur von 140 °C in einen Trioxanreaktor, der 300 ml einer siedenden Formaldehydlösung mit
etwa 50 Gew.% Formaldehyd,
etwa 15 Gew.% Schwefelsäure
enthielt, mit einer Geschwindigkeit von 100 g/l (bezogen auf Formalin) eingeleitet, so daß die Lösung ohne weitere Wärmezufuhr am Sieden gehalten wurde.

Die dabei erzielte Trioxankonzentration im Synthesedampf betrug 32 Gew.%, was einem Formaldehydumsatz pro Reaktordurchgang von nahezu 52 % entspricht.

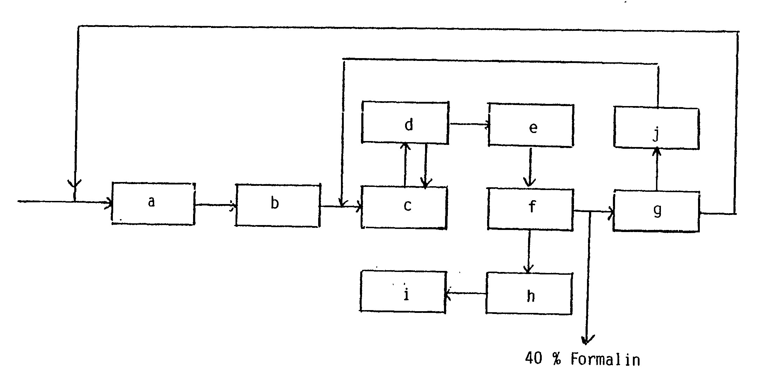

## Patentansprüche

1. Verfahren zur Herstellung von Trioxan aus gasförmigem, erhitztem Formaldehyd durch Trimerisierung in Gegenwart eines Säurekatalysators, und Isolation des Trioxans aus dem Kondensat durch Extraktion mit einem mit Wasser nicht mischbaren inerten Lösungsmittel und Destillation dadurch gekennzeichnet, daß man einen heißen, eine Temperatur von 110°-140 °C aufweisenden Formaldehyd enthaltenden Gasstrom in den unteren Teil des Trioxanreaktors einleitet, wobei die in dem Reaktor enthaltene etwa 30 bis 70 Gew.%ige Formalinlösung, die einen Säurekatalysator enthält und einen pH-Wert aufweist, der dem von 2 bis 25 Gew.% Schwefelsäure entspricht, auf den Siedepunkt erhitzt und gut durchgemischt wird, den aus dem Trioxanreaktor austretenden Trioxan, Formaldehyd und Wasser enthaltenden Synthesedampf an einem, dem Trioxanreaktor unmittelbar nachgeordneten Dephlegmator, der mit Wasser auf etwa 90 °C gekühlt wird, in eine an Formaldehyd reiche Phase, die in den Trioxanreaktor zurückgeführt wird, und in eine an Trioxan reiche Phase, die kondensiert wird, unterteilt und das Trioxan enthaltende Kondensat weiterverarbeitet.

2. Verfahren nach Anspruch 1, dadurch gekennzeichnet, daß man als Formaldehydlieferanten den aus der katalytischen Methanoloxidation stammenden Formaldehyd enthaltenden Gasstrom verwendet.

3. Verfahren nach Anspruch 2, dadurch gekennzeichnet, daß man als Formaldehydlieferanten den aus dem Formox-Verfahren stammenden, Formaldehyd enthaltenden Gasstrom verwendet.

4. Verfahren nach Anspruch 2, dadurch gekennzeichnet, daß man als Formaldehydlieferanten den aus dem Silberkontaktverfahren stammenden, Formaldehyd enthaltenden Gasstrom verwendet.

## Claims

1. Process for the preparation of trioxan from gaseous, heated formaldehyde by means of trimerisation in the presence of an acid catalyst, and isolation of the trioxan from the condensate by extraction with an inert, water-immiscible solvent, followed by distillation, characterised in that a hot formaldehyde-containing stream of gas, at a temperature of 110-140 °C, is introduced into the lower part of the trioxan reactor, whereby the approximately 30 to 70 % by weight formalin solution in the reactor, containing an acid catalyst and with a pH corresponding to that of 2 to 25 % by weight of sulphuric acid, is heated to its boiling point and is well mixed ; the trioxan, formaldehyde and water-containing steam resulting from the synthesis is removed from the trioxan reactor and separated, by means of a reflux condenser which is installed immediately next to the trioxan reactor and cooled with water to about 90 °C, into a formaldehyde-rich phase, which is returned to the trioxan reactor, and a trioxan-rich phase, which is condensed and further processed.

2. Process according to claim 1, characterised in that a formaldehyde-containing stream of gas from the catalytic oxidation of methanol is used as a source of formaldehyde.

3. Process according to claim 2, characterised in that a formaldehyde-containing stream of gas produced in the Formox process is used as a source of formaldehyde.

4. Process according to claim 2, characterized in that a formaldehyde-containing stream of gas produced in the silver contact method is used as a source of formaldehyde.

## Revendications

1. Procédé de préparation du trioxanne à partir du formaldéhyde gazeux chaud par trimérisation en présence d'un catalyseur acide et isolement du trioxanne à partir du condensat par extraction à l'aide d'un solvant inerte non miscible à l'eau et distillation, caractérisé en ce que l'on envoie un courant de gaz chaud, à une température de 110 à 140 °C, contenant du formaldéhyde, dans la partie inférieure du réacteur à tioxanne, on chauffe au point d'ébullition et on mélange soigneusement la solution de formaldéhyde à une concentration d'environ 30 à 70 % en poids qui se trouve dans le réacteur, qui contient un catalyseur acide et présente un pH correspondant à celui de l'acide sulfurique à une concentration de 2 à 25 % en poids, on sépare la vapeur de synthèse sortant du réacteur à trioxanne, qui contient du trioxanne, du formaldéhyde et de l'eau, dans un déphlegmateur placé immédiatement derrière le réacteur à trioxanne et qui est refroidi à 90 °C environ par de l'eau, en une phase riche en formaldéhyde, qui est recyclée au réacteur à trioxanne, et en une phase riche en trioxanne, qui est condensée, puis on soumet le condensat contenant le trioxanne aux opérations d'isolement.

2. Procédé selon la revendication 1, caractérisé en ce que l'on utilise en tant que matière première apportant le formaldéhyde le courant de gaz contenant du formaldéhyde provenant de l'oxydation catalytique du méthanol.

3. Procédé selon la revendication 2, caractérisé en ce que l'on utilise en tant que matière première apportant le formaldéhyde le courant de gaz contenant du formaldéhyde provenant du procédé Formox.

4. Procédé selon la revendication 3, caractérisé en ce que l'on utilise en tant que matière première apportant le formaldéhyde le courant de gaz contenant du formaldéhyde provenant du procédé par catalyse à l'argent.

a

b

c

d

e

f

g

h

i

j

40 % Formalin